# EUROPEAN PATENT SPECIFICATION

(11) **EP 1 539 075 B1**
(45) Date of publication and mention of the grant of the patent: **04.01.2006**
(21) Application number: 03791525.3
(22) Date of filing: 27.08.2003
(51) Int. Cl.: A61F 13/56, A61F 13/471

(54) **FIXING OF MALE INCONTINENCE PAD IN UNDERGARMENTS**
FIXIERUNG EINER EINLAGE GEGEN INKONTINENZ DES MANNES IN UNTERWÄSCHE
FIXATION DE SERVIETTE POUR HOMME INCONTINENT DANS LES SOUS-VETEMENTS

(30) Priority: 30.08.2002 SE 0202566
(43) Date of publication of application: 15.06.2005
(73) Proprietor: SCA Hygiene Products AB, 405 03 Göteborg (SE)
(72) Inventor: HANSSON, Roy, S-431 50 Mölndal (SE); HERMANSSON, Sofia, S-426 71 Västra Frölunda (SE)
(74) Representative: Romare, Laila Anette
(86) International application number: PCT/SE2003/001323
(87) International publication number: WO 2004/019850

(56) References cited:
- DE-U1- 29 602 160
- US-A- 5 435 014
- US-A- 5 716 350
- DATABASE WPI Week 200008, Derwent Publications Ltd., London, GB; Class D22, AN 2000-090558, XP002903021 & JP 11 318 985 A (SHISEIDO CO. LTD.)

## Description

The invention relates to a disposable incontinence pad for men.

The incontinence pad has a front end portion and comprises an absorbent part with an upper liquid-permeable surface layer, a lower backing layer, and an absorbent body arranged between the liquid-permeable surface layer and the backing layer.

The incontinence pad is also provided with a fastening system for fixing the incontinence pad to a pair of underpants, fixing pants or the like.

### BACKGROUND ART

What is often referred to as mild incontinence and which means leakage of small quantities of urine is not perceived as a mild inconvenience by the person who is affected. Mild incontinence is a hidden handicap from which many people suffer. Men with prostate trouble constitute a large group of mildly incontinent men. After prostate surgery, many men suffer from incontinence dribbling, which has hitherto led to mental suffering for many of them because suitable incontinence protection has not been available.

Incontinence pads used hitherto for men suffering from mild incontinence are characterized in that they are often constructed in the same way as incontinence pads intended for women suffering from milder forms of incontinence. Use is often even made of the same types of incontinence pad for both men and women. This is due to the fact that mild incontinence is more common among women than among men, for which reason incontinence pads are specially adapted to the anatomy of women and are therefore by no means particularly suitable for the anatomy of men.

Mildly incontinent men often also use special incontinence pads intended for heavier forms of incontinence. These incontinence pads are normally large and awkward to wear for men who do not have any problems other than their incontinence and therefore wish to function entirely normally in society.
These incontinence pads are moreover uncomfortable to wear and far from inconspicuous under normal clothes.
Special incontinence pads intended for men with mild incontinence are previously known per se.
One type of such known pads encloses the male genitals in a Kind of cone-shaped container during use. Disadvantages of this type of incontinence pads are that they are altogether too warm and enclosed and thus uncomfortable for the user to wear. By virtue of their construction, another disadvantage is that the pads are too rigid to be comfortable. The fixing of the pads to the body of the wearer or to the underpants or special fixing pants of the wearer is also an area where effective solutions are lacking for these types of protection.
One type of incontinence pad for men is known from Swedish patent document SE 450 811. The pad consists of an upper shield-like part which, during use of the pad, lies close over the penis and scrotum, and a lower part which, during use of the pad, curves in under the penis and scrotum of the wearer without entirely surrounding these. The pad has a downwardly narrowing, bowl-like shape. With such a pad, close enclosure of the genitals of the wearer is therefore avoided, which is of course an advantage from the point of view of comfort. The pad is held in place by means of double adhesive tape, arranged on that side of the pad which faces away from the wearer during use, the double adhesive tape being arranged so that fixing takes place to the underpants or special fixing pants of the wearer in the crotch portion.
In another patent document, SE 500793, an incontinence pad is described, which also has a downwardly narrowing, bowl-like shape. The pad, which in the extended state has the shape of a triangle, comprises a liquid-permeable surface layer arranged on that side of the pad which faces the wearer during use, a liquid-impermeable surface layer on the opposite side, and an absorption body arranged between the surface layers. Arranged between the surface layers along the two essentially longitudinal edges of the pad are elastic elements, by virtue of which the product acquires a curved bowl-shape when the elastic elements contract from the stretched state they are in when manufactured. The document does not describe how the pad is fixed to the wearer or the underpants of the wearer during use, but it can be assumed that the pad is Intended to be used together with relatively close-fitting underpants, the pad being expected to be held in place by the underpants without any special fixing means or by some form of fastening means such as, for example, pressure-sensitive adhesive or hook and loop means attached to the underpants of the wearer in the crotch portion.

Swedish patent specification SE 508240 describes an incontinence pad for men, which has an initially rectangular material piece comprising a liquid-permeable surface layer arranged on that side of the pad which faces the wearer during use, a liquid-impermeable surface layer on the opposite side, and an absorption body arranged between the surface layers. In manufacture, the material piece is Z-folded along its two longitudinal edges and then sealed in a liquidtight manner along the rear transverse edge.
During use of the pad, the Z-folded edge areas which have not been sealed are drawn apart, the sealing along the rear transverse edge holding the pad together in its rear end portion. The pad then has a downwardly narrowing, bowl-like shape. The pad is held in place by means of hook and loop material, arranged on that side of the pad which faces away from the wearer during use, the hook and loop material being arranged so that fixing takes place to the underpants of the wearer in the crotch portion.
JP11318985 and US 5 435 014 disclose absorbent articles having protruding fasteners at both ends. Such articles are perceived by male users as being very uncomfortable since they extend rearward beyond the crotch region of the user and cause unnecessary chafing and pressure and restrict natural movement of the male genitals.
Another incontinence pad for men is described in Swedish patent document SE 449172. The document describes an incontinence pad for men suffering from milder forms of incontinence. The pad is designed as an absorbent collecting bag, the opening of the bag comprising a cutout In that part of the bag which is intended to face the wearer during use. During use of the pad, the cutout is to be positioned below the scrotum of the wearer so that the scrotum is placed inside the collecting bag together with the penis. The pad is fixed by virtue of being fastened around the scrotum and penis of the wearer during use.
Users of mild incontinence protection for men are predominantly older men because incontinence problems are mainly age-related. Many older men prefer underpants or fixing pants which are relatively voluminous in the crotch portion, and therefore dislike underpants or fixing pants which are close-fitting.
Incontinence pads which are fixed to the underpants of the wearer in the crotch portion adapt to the crotch position of the fixing pants during use. In the case of fixing to underpants or fixing pants with voluminous crotch portions, this means that the incontinence pad takes up a position a small distance away from the genitals of the wearer, and also that the incontinence pad tends to move relative to the genitals of the wearer when the wearer moves. An incontinence pad which does not lie closely against the genitals of the wearer during urination involves a significantly increased risk of leakage. A problem of comfort is also associated with an incontinence pad which can move in a more or less uncontrolled manner relative to the body of the wearer during use.

Incontinence pads for men are also used by some older men who, owing to senility for example, have lost their capacity to control some of their bodily functions, inter alia their capacity to control their discharge of urine. One problem associated with the use by these senile older men of incontinence pads which are fixed to the crotch portion of the underpants is that the men, when they attempt to urinate in a normal manner, have to pull down their underpants or fixing pants because the incontinence pad covers the fly opening of the underpants. The underpants are then pulled down to knee height, after which urination is performed. Urination is often not particularly successful, but a certain amount of urine strikes and wets the front part of the underpants situated between the front edge of the incontinence pad and the waistband of the underpants.

Incontinence pads which are intended to be fixed by virtue of being fastened around the genitals of the wearer have the disadvantage that they are fastened either too tightly and are therefore extremely uncomfortable to wear or too loosely and therefore come away from the genitals, the function of the pad thus being lost completely. These types of incontinence pad are also usually very warm and uncomfortable to wear.

Warm and uncomfortable articles of clothing, incontinence pads and the like are a major problem as far as the care of senile older men is concemed. It is quite common for these men, unawares, to attempt to remove these uncomfortable articles of clothing or incontinence pads from the body. An uncomfortable and poorly fixed incontinence pad is then an article which a senile person often attempts to get rid of.

A need therefore remains for an improved fixing system for a mild incontinence pad for men which both makes the pad comfortable to wear and effectively holds the pad in place against the genitals of the wearer throughout the period of use, irrespective of whether the wearer of the incontinence pad prefers underpants or fixing pants with a voluminous or close-fitting crotch portion.

A need also remains for an incontinence pad for men which is so comfortable to wear that a senile wearer does not unawares attempt to remove the incontinence pad from the body.

A need moreover remains for an incontinence pad which protects the underpants of the wearer from being wetted by urine when the wearer urinates while standing with his underpants pulled down.

### DISCLOSURE OF INVENTION

By means of the present invention, however, an article of the kind referred to in the introduction has been produced, which article essentially eliminates the problems associated with previously known mild incontinence pads for male users.

In this connection, an incontinence pad of the kind referred to in the introduction is characterized mainly in that the fastening system of the incontinence pad comprises at least one fixing tongue extending in the longitudinal direction of the incontinence pad from the front end portion.

By virtue of this, an incontinence pad is obtained which is fixed to the underpants of the wearer above the crotch portion of the underpants. The incontinence pad is then not forced to adapt to the crotch portion of the underpants of the wearer, but can instead be held in the correct position in relation to the genitals of the wearer. By virtue of the fact that the securing position of the fixing tongue, and thus the position of the entire incontinence pad, is adjustable in the vertical direction on fitting, optimum adaptation of the absorbent part of the incontinence pad in relation to the genitals of the wearer is made possible. After adjustment of the incontinence pad, the fixing tongue is fixed to the underpants or fixing pants of the wearer.

In an incontinence pad which is especially easy to manufacture, the fixing tongue comprises an extension of at least one of the upper, liquid-permeable surface layer and the lower backing layer.

According to one embodiment of the invention, the fixing tongue can comprise a thin absorbent material layer.

The incontinence pad can also be provided with a fastening means arranged on the fixing tongue. The fastening means is intended to be fixed to the undergarments of the wearer during use. The fastening means of the incontinence pad can then be arranged so that it can be fixed to the inside of the underpants of the wearer in the area between the crotch portion and the waist of the underpants.

Alternatively, the incontinence pad can comprise a longer fixing tongue intended to be folded around the waist of the underpants, fixing pants or the like of the wearer. The waist elastic of the underpants then fixes the incontinence pad via the fixing tongue. In order to strengthen the waist elastic fixing of the incontinence pad, a fastening means can be arranged on the fixing tongue, the fastening means being intended to be fixed to the outside of the fixing pants.

An incontinence pad according to a further embodiment of the invention is especially advantageous with regard to packing the incontinence pad. In this connection, the incontinence pad comprises a fixing tongue having a size and shape which are such that the entire fixing tongue is accommodated inside the edges of the absorbent part of the incontinence pad when the entire fixing tongue is folded over the liquid-permeable surface layer or the backing layer. The incontinence pad is then easy to fold further and pack in a separate case or together with other incontinence pads directly in a consumer pack.

An incontinence pad according to still another embodiment comprises a fixing tongue which has an essentially triangular shape with one of the bases of the triangle connected to the absorbent part of the incontinence pad, so that the fixing tongue narrows forwards in the longitudinal direction of the incontinence pad. That part of the fixing tongue which is folded around the waist of the underpants and is fixed by the waist elastic during use is then considerably smaller and more discreet.

The fixing tongue of the incontinence pad can consist of a band. It is also possible to provide the incontinence pad with two fixing tongues.

A fastening means arranged on the fixing tongue can comprise a pressure-sensitive adhesive surface. A removable protective layer is then arranged over the pressure-sensitive adhesive surface, that surface of the protective layer which is arranged away from the pressure-sensitive adhesive surface advantageously being connected to the backing layer of the incontinence pad.

An incontinence pad according to the last-mentioned embodiment can alternatively have at least one area of the backing layer which is treated with a release agent. The pressure-sensitive adhesive surface of the fastening means is then connected removably to the release-agent-treated area of the backing layer.

The incontinence pad can also have a fastening means which comprises a hook and loop surface, a removable protective layer being arranged over the hook and loop surface. The protective layer is then advantageously connected to the backing layer of the incontinence pad.

The incontinence pad according to a fifth embodiment of the invention has a fixing tongue which can be separated from the absorbent part of the incontinence pad.

The incontinence pad can also comprise a second fastening means, the second fastening means being arranged on the backing layer of the absorbent part. A removable protective layer is then suitably arranged over the second fastening means. For use of the incontinence pad, the wearer can then choose whether he wants to make use of the second fastening means by removing the protective layer and in so doing activating the second fastening means, or opt not to use the second fastening means and then allow the protective layer to remain over the fastening means.

In order to adapt the incontinence pad to the male anatomy in the best way, the absorbent part of the incontinence pad has, according to one embodiment, its greatest extent in the transverse direction in the part which, in the longitudinal direction, is located towards the fixing tongue and its smallest extent in the part which, in the longitudinal direction, is located away from the fixing tongue. An incontinence pad according to the invention therefore preferably has a shape which narrows in the direction from the front part to the rear part. The front part of the incontinence pad will thereby be delimited by the fixing tongue, and the rear part by the absorbent part, which rear part is intended to be placed over the user's genitals.

### BRIEF DESCRIPTION OF FIGURES

The invention will be described in greater detail below with reference to the illustrative embodiments shown in the accompanying figures, in which:

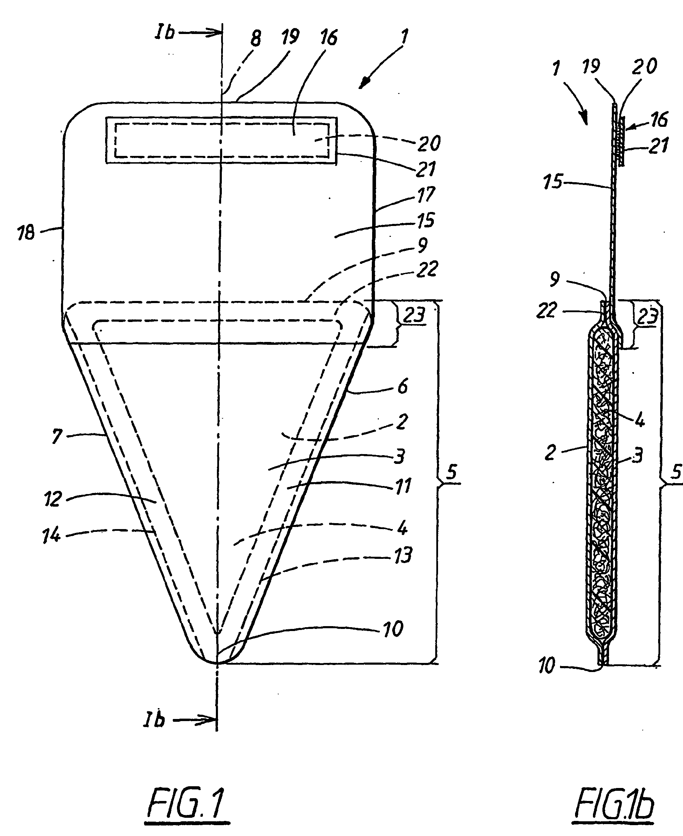

- Figure 1: shows a first embodiment of an incontinence pad according to the invention in an extended state, from the side intended to face away from the wearer during use;
- Figure 1 b: shows a section through the incontinence pad, along the line lb-lb, in Figure 1;
- Figure 2: shows a perspective view of the incontinence pad according to Figure 1 with active contracted elastic, from the side intended to face the wearer during use;
- Figure 3: shows a second embodiment of an incontinence pad according to the invention with active contracted elastic and seen from the side intended to face the wearer during use;
- Figure 4: shows a third embodiment of an incontinence pad according to the invention with active contracted elastic and seen from the side intended to face the wearer during use;
- Figure 5: shows a fourth embodiment of an incontinence pad according to the invention in a packing configuration;
- Figure 6: shows the incontinence pad according to Figure 5 in a configuration immediately before fitting in the undergarments of a wearer and seen from the side intended to face away from the wearer during use, and
- Figure 7: shows a fifth embodiment of an incontinence pad according to the invention.

### DESCRIPTION OF EMBODIMENTS

The invention relates to a mild incontinence pad for men.

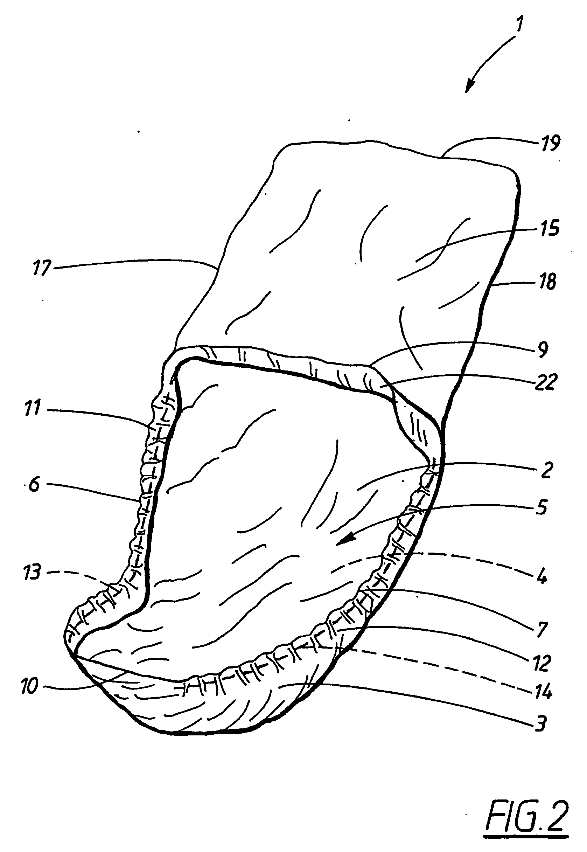

The first embodiment shown in Figures 1, 1 b and 2 relates to an incontinence pad 1 for mildly incontinent men. Figure 1 shows the incontinence pad 1 in an extended state, seen from the side intended to face away from the wearer during use, and Figure 2 shows the incontinence pad 1 in a use-like contracted configuration from the side intended to face the wearer during use. Figure 1 b shows a section through the incontinence pad 1 along the line Ib-Ib in Figure 1.

The incontinence pad 1 comprises an absorbent part 5. The absorbent part 5 comprises a liquid-permeable surface layer 2, a backing layer 3, and an absorption body 4 enclosed between the surface layer 2 and the backing layer 3.

The absorbent part 5 of the incontinence pad 1 has the shape essentially of an isosceles triangle, the two edges 6, 7 of the same length extending essentially in the same direction as the longitudinal symmetry line 8 of the incontinence pad 1. The third edge constitutes the front edge 9 of the absorbent part 5, the front edge 9 extending transversely to the longitudinal symmetry line 8 of the incontinence pad 1. That area of the absorbent part 5 which is located towards the front edge 9 constitutes the front end portion 23 of the absorbent part 5 and is intended to face forwards on the wearer during use. The triangle point 10, which is delimited by the two edges 6, 7 of the absorbent part 5 of the incontinence pad 1, constitutes the rear part of the incontinence pad and is intended to face backwards on the wearer during use and surround the genitals of the wearer. The absorbent part 5 has its greatest extent in the transverse direction in the area which faces forwards on the wearer during use.

The absorption body 4 can be constructed from one or more layers of cellulose fluff pulp. The cellulose fluff pulp can then be mixed with fibres or particles of a highly absorbent polymer material of the kind which, during absorption, chemically binds great quantities of liquid, while forming a liquid-containing gel. The absorption body 4 can also comprise highly absorbent polymer material arranged in one or more layers inside the absorption body or adjacent to the surface(s) of the absorption body. The absorption body 4 can also include components of a non-absorbent nature in order to improve the characteristics of the absorption body 4. Examples of such components are binding fibres, shape stabilizing components, reinforcing fibres or the like. Various types of layer and material provided for improved spreading or liquid can also form part of the absorption body 4. The absorption body 4 can of course also consist of other types of absorption material, such as absorbent nonwoven material, absorbent foam, textile materials, peat, or mixtures of different kinds of absorption material. Special layers for rapidly receiving large quantities of liquid and temporarily storing this liquid, subsequently conveying the temporarily stored liquid to other parts of the absorption body 4, can also be included in incontinence pads of the type specified. Such receiving layers are then normally arranged between the liquid-permeable covering layer 2 and the absorption body 4 of the incontinence pad 1. No receiving layer is illustrated in any of the figures.

The liquid-permeable surface layer 2 extends outside the absorption body 4 along the entire periphery of the absorption body 4. The liquid-permeable surface layer 2 can consist of any material suitable for the purpose. Examples of common liquid-permeable covering materials are nonwoven textile materials, perforated plastic films, net made of plastic or textile, and liquid-permeable foam layers or the like. Liquid-permeable surface layers which consist of continuous thin fibres extending fundamentally in the longitudinal or transverse direction of the article are also found. Laminates consisting of two or more of the possible covering materials mentioned above are also common, as are coverings consisting of different materials in different parts of the surface.

The backing layer 3 also extends outside the absorption body 4 along the entire periphery of the absorption body 4. The backing layer 3 also can consist of a number of different materials. It is most common for the backing layer 3 to consist of a thin liquid-impermeable plastic film, but it is also possible to use other types of liquid-impermeable material, such as nonwoven materials which have been rendered liquid-impermeable by, for example, plastic coating, liquid-impermeable foam layers, liquid-impermeable adhesive or the like. The liquid-impermeable backing layer 3 can also consist of a vapour-permeable material. Laminates comprising a liquid-impermeable layer arranged towards the absorption body and a more textile-like layer arranged away from the absorption body are also common as a backing layer 3.

The liquid-permeable surface layer 2 and the backing layer 3 are interconnected outside the edges of the absorption body 4 along the entire periphery of the absorption body 4 and constitute side flaps 11, 12 in the edge areas 6, 7 and a front flap 22 along the front edge 9. The liquid-permeable surface layer 2 and the backing layer 3 can be interconnected in a number of different ways. Examples of connection methods are gluing, hot-melting, ultrasonic welding or the like.

Elastic elements 13, 14 are arranged in a prestressed manner in the essentially longitudinal side flaps 11, 12 of the same length. The function of the elastic elements 13, 14 is to curve the absorbent part 5 of the incontinence pad 1 into a bowl-shape during use. The elastic elements moreover raise the essentially longitudinal side flaps 11, 12 up from the plane of the incontinence pad 1, by virtue of which liquid is prevented from running over the edges 6, 7 of the incontinence pad 1, and in this way form liquid barriers together with surrounding layers. The elastic elements 13, 14 consist of one or more elastic threads which have been applied in a stretched state between the liquid-permeable surface layer 2 and the backing layer 3. The elastic elements 13, 14 are connected to the backing layer 3 and the surface layer 2 by gluing, ultrasonic welding, thermal welding or the like.

In alternative embodiments, the elastic elements 13, 14 can be arranged on that side of the side flaps 11, 12 intended to face the wearer during use, or on the opposite side of the side flaps, and are then of course connected to only the surface layer 2 or, respectively, the backing layer 3.

In alternative embodiments, the elastic elements 13, 14 can consist of elastic band material made of, for example, foamed material.

The incontinence pad 1 is characterized mainly in that it comprises a fixing tongue 15 arranged in the extension of the absorbent part 5 of the incontinence pad 1 towards the front.

The fixing tongue 15 has longitudinal edges 17, 18 and a front transverse edge 19. The fixing tongue 15 consists of a flexible piece of material, which is suitably air-permeable so as to obtain optimum comfort characteristics. The piece of material can consist of any material suitable for the purpose. Examples of usable materials are nonwoven textile materials, thin plastic films which are preferably air-permeable, net made of plastic or textile, foam layers or the like. Laminates consisting of two or more of the materials mentioned above are also commonly found. The fixing tongue 15 is connected to the front end portion 23 of the absorbent part 5 by means of gluing, ultrasonic welding or the like.

The fixing tongue 15 advantageously consists of an extension of the liquid-permeable surface layer 2 or the backing layer 3 or of both layers 2, 3, that is to say an extension of the front flap 22.

The fixing tongue 15 can also comprise a thin flexible absorbent material layer such as, for example, a tissue layer, a thin layer of cellulose fluff pulp or the like. The absorbent layer is then capable of absorbing small amounts of urine which may strike the fixing tongue 15 when a wearer urinates in the conventional manner, that is to say standing in front of a toilet or the like, the wearer having pulled his underpants or fixing pants down to knee height.

A fastening means 16 is suitably arranged on the fixing tongue 15. The fastening means 16 comprises a rectangular adhesive surface 20, the long sides of which extend in the transverse direction of the incontinence pad 1. A removable protective layer 21 is arranged over the adhesive surface 20, the protective layer 21 being removed immediately before fixing of the adhesive surface 20 to the underpants or fixing pants of the wearer. The adhesive surface 20 is intended to be fixed to the underpants of the wearer on that side of the underpants facing the wearer during use, that is to say to the inside of the underpants. The adhesive surface 20 is fixed to the front part of the underpants in the area between the waist elastic and the crotch portion of the underpants, that is to say in that area of the underpants of which the position is determined by the design of the underpants and by the location the wearer chooses for the waist of the underpants. The position of the absorbent part 5 of the incontinence pad 1 in relation to the wearer is adjusted on fixing to the underpants by the fixing tongue 15 being adjusted in the vertical direction immediately before the adhesive surface 20 is fixed to the inside of the underpants. The fixing principle means that the position of the incontinence pad 1 is not dependent on whether the wearer prefers underpants with a voluminous or close-fitting crotch portion, but only on where on the underpants the adhesive surface 20 is positioned.

Other types of fastening means 16 are also conceivable such as, for example, surfaces which consist of high-friction material. In this case, the incontinence pad 1 is held in place during use by virtue of the fastening means 16 being fastened between the underpants and the abdomen of the wearer. Surfaces made of hook and loop material or the like for fixing to the inside of the underpants are also conceivable as fastening means 16.

It is also conceivable to design the fixing tongue 15 without any fastening means 16. The fixing means 15 should then have sufficient length in order to reach over and be folded around the waist of the underpants, so that the incontinence pad 1 is held in place by virtue of the waist elastic of the underpants retaining the fixing tongue 15 in its position during use of the incontinence pad 1.

A combination of the two fixing methods described above is also conceivable, that is to say a fixing tongue 15 both of sufficient length to be capable of being folded around the waist edge of the underpants and with a fastening means 16 which is fixed to the underpants. The fastening means 16 suitably consists of an adhesive surface 20 or the like. The adhesive surface 20 can then be arranged so close to the front transverse edge 19 of the fixing tongue 15 that the adhesive surface 20 comes to lie against the outside of the underpants after folding of the fixing tongue 15 around the waist of the underpants. Alternatively, the adhesive surface 20 can be placed closer to the absorbent part 5 of the incontinence pad 1 on the fixing tongue 15, the adhesive surface 20 coming to lie between the area where the fixing tongue 15 is folded around the waist of the fixing pants and the absorbent part of the incontinence pad 1. Fixing of the adhesive surface 20 to the underpants will then take place on the inside of the underpants between the waist and the crotch portion of the underpants. A further alternative is for the adhesive surface 20 to be positioned on the fixing tongue 15 so that the adhesive surface 20 comes to lie where the fixing tongue 15 is folded around the waist of the underpants, fixing of the adhesive surface 20 taking place on both sides of the underpants.

The fixing tongue 15 can be provided with a number of adhesive surfaces 20, at least one adhesive surface 20 being intended to be fixed to the inside of the underpants, and at least one adhesive surface 20 being intended to be fixed to the outside of the underpants. The cut of the waist of the underpants and how the wearer chooses to place the waist of the underpants on the body of course influence whether the adhesive surface 20 is fixed to the outside or the inside of the underpants. Fixing of the adhesive surface 20 to the outside or the inside of the underpants does not, however, have any decisive significance for the position of the absorbent part 5 of the incontinence pad 1 during use because the position of the incontinence pad 1 in relation to the genitals of the wearer is determined only by the adjustment described above of the incontinence pad 1 in connection with fitting.

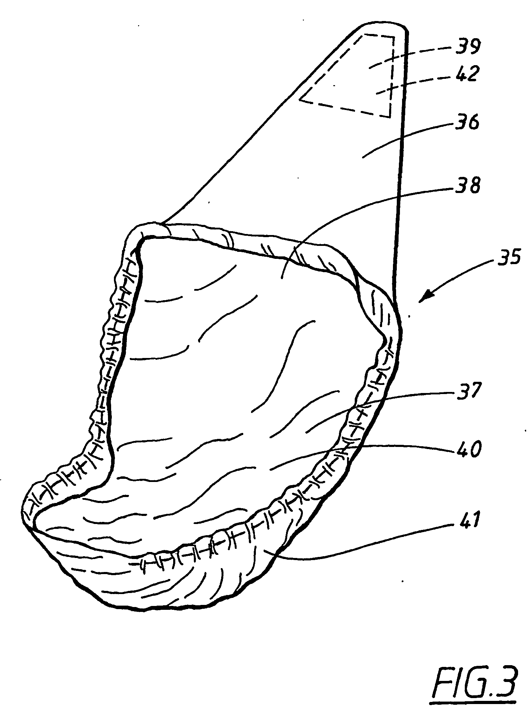

Figure 3 shows a second embodiment of the invention. The figure shows the incontinence pad 35 in a use-like contracted configuration from the side intended to face the wearer during use.

The incontinence pad 35 has a fixing tongue 36 arranged in the extension of the absorbent part 37 of the incontinence pad 35 towards the front. The fixing tongue 36 has an essentially triangular shape with a base connected to the front portion 38 of the absorbent part 37. The fixing tongue 36 narrows in the direction away from the front portion 38 of the absorbent part 37.

A fastening means 42 comprising an adhesive surface 39, for fixing the incontinence pad 35 to the undergarment of a wearer, is arranged on the fixing tongue 36 at the end far from the front portion 38 of the absorbent part 37. The adhesive surface 39 also has an essentially triangular shape.

The length of the fixing tongue 36 can be adapted for fixing the adhesive surface 39 to the inside of the underpants or for being folded around the waist of the underpants so that the adhesive surface is intended to be fixed to the outside of the fixing pants.

One advantage of a concentrated adhesive surface 39 on the fixing tongue 36 of, for example, triangular shape is that it is easier to fix the more concentrated adhesive surface 39 to the undergarment using only one hand than it is to fix a more extended surface. On the whole, it is sufficient to fix the adhesive surface 39 to the undergarments by applying a light concentrated pressure over the concentrated adhesive surface 39 when the latter is correctly positioned against the surface of the undergarment. Other shapes of concentrated adhesive surfaces 39 such as circular, square or the like are also conceivable.

Another advantage of an incontinence pad 35 which has a triangular fixing tongue 36 according to Figure 3 is that that part of the fixing tongue 36 which may be folded around the waist of the underpants on fitting is narrower and thus also more discreet.

A third advantage of a fixing tongue 36 according to the embodiment is that, as far as packing the incontinence pad 35 is concerned, the fixing tongue 36 has a shape which is adapted to the shape of the absorbent part 37 of the incontinence pad 35. On packing of the incontinence pad, the entire fixing tongue 36 is then, as a first step, folded in over the liquid-permeable surface layer 40 or the backing layer 41. The entire fixing tongue 36 will therefore, on account of its shape adapted to the absorbent part 37 of the incontinence pad 35, be accommodated inside the edges of the absorbent part 37 after folding of the fixing tongue 36. The incontinence pad 35 is therefore particularly easy to pack or fold more times before packing.

Other shapes of the fixing tongue 36, designed so that the entire fixing tongue comes to lie inside the edges of the absorbent part 37 on folding of the fixing tongue 36 over the liquid-permeable surface layer 40 or the backing layer 41, are also conceivable. For example, the fixing tongue 36 can consist of a narrow band or string projecting from the front portion 38 of the absorbent part 37. The band or string then extends essentially along the longitudinal symmetry line of the incontinence pad 35. The band or string can be fixed to the underpants of the wearer by being folded around the waist of the underpants or by virtue of the ends of the band or string having been provided with fastening means 42.

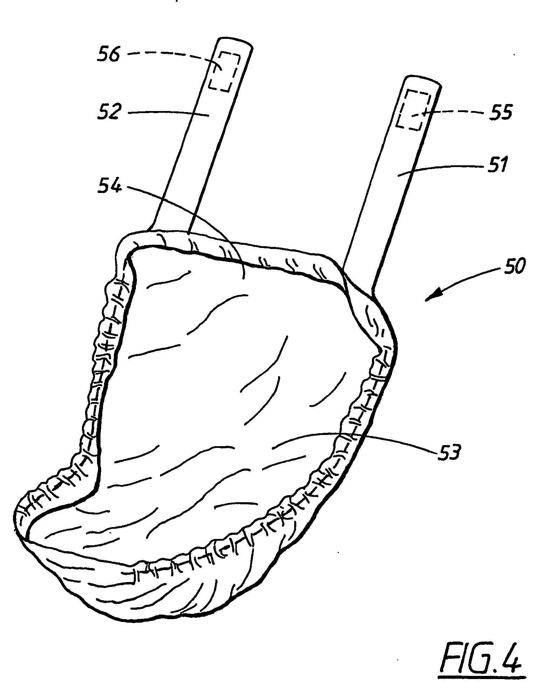

Figure 4 shows a third embodiment of an incontinence pad 50 according to the invention. The figure shows the incontinence pad 50 from the side intended to face the wearer during use.

The incontinence pad 50 has double fixing tongues 51, 52 arranged in the extension of the absorbent part 53 of the incontinence pad 50 towards the front. The fixing tongues 51, 52 are connected to the front portion 54 of the absorbent part 53 and extend essentially parallel to the longitudinal symmetry line of the incontinence pad 50. The fixing tongues 51, 52 are each arranged on their own side and essentially at the same distance from the longitudinal symmetry line of the incontinence pad 50. The fixing tongues 51, 52 have fastening means 55, 56 arranged on the fixing tongues 51, 52 at the end far from the front edge 54 of the absorbent part 53. The lengths of the fixing tongues 51, 52 can be adapted for fixing to the inside of the underpants or adapted so as to be folded around the waist of the underpants.

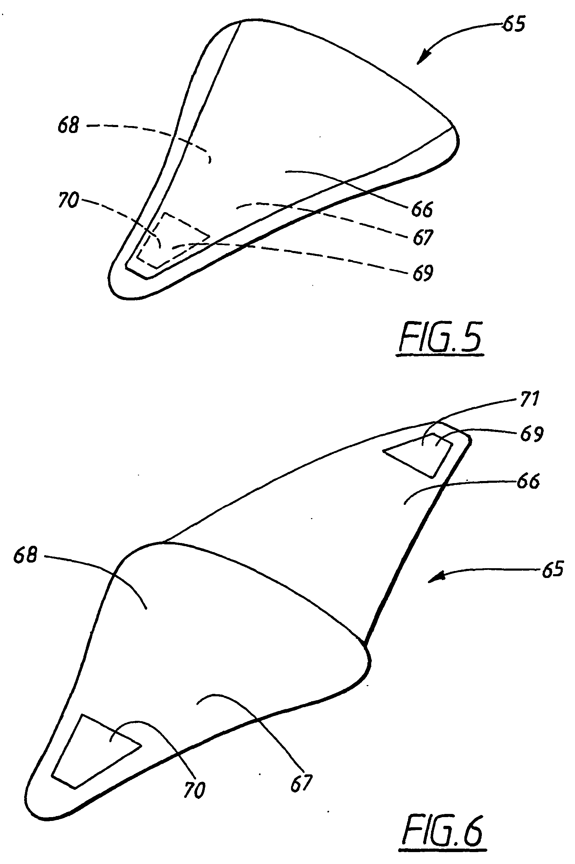

Figures 5 and 6 show a fourth embodiment of the invention. Figure 5 shows the incontinence pad 65 when it is taken out of its packing before fitting, when the fixing tongue 66 is folded over the backing layer 68 of the absorbent part 67 of the incontinence pad 65.

In Figure 6, the fixing tongue 66 is folded out before fitting in the underpants of a wearer**.**

The incontinence pad 65 according to the embodiment comprises a fastening means 69 intended for fixing the fixing tongue 66 to the underpants of the wearer. The fastening means 69 comprises a pressure-sensitive adhesive surface 71. A protective layer 70 is preferably permanently connected to the backing layer 68 and removably connected to the adhesive surface 71 of the fastening means 69 when the fixing tongue 66 is folded over the backing layer 68 of the incontinence pad 65. The protective layer 70 consists of a material piece made of paper, the protective layer 70 being release-agent-treated on the side facing the fastening means 69 when the fixing tongue 66 is folded over the absorbent part 67 of the incontinence pad 65. The non-release-agent-treated second side of the protective layer 70 is suitably permanently connected to the backing layer by means of adhesive or the like.

The protective layer 70 can also consist of other suitable materials such as a plastic layer or the like.

Release-agent treatment of the protective layer 70 preferably means that the layer is provided with a thin silicone layer, a wax layer or the like. It is also possible to provide a protective layer 70 which is removable from the pressure-sensitive adhesive surface 71 by arranging raised portions on the surface of the protective layer 70, by means of embossing for example. The contact surface between the pressure-sensitive adhesive surface 71 and the protective layer 70 is then so small that the adhesive layer is removable.

It is also possible for the separate protective layer 70 to be omitted, at least that area of the backing layer 68 which is in contact with the fastening means 69 when the fixing tongue 66 is folded over the backing layer 68 of the absorbent part 67 of the incontinence pad 65 instead being treated with release agent. Treatment of the backing layer 68 with release agent is then carried out according to the same principles as release-agent treatment of a protective layer, that is to say siliconization, waxing, embossing or the like.

Embodiments in which the fastening means 69 consists of high-fridion material, hook and loop material or the like are also conceivable. If the fastening means 69 consists of high-friction material, it is not necessary to provide a separate protective layer.

In cases where the fastening means 69 consists of hook and loop material, that side of the protective layer 70 which faces the fastening means 69 must consist of a suitable material which adheres removably to the hook and loop material of the fastening means 69. The opposite side of the protective layer 70 must be designed so that it can be connected to the backing layer 68.

An incontinence pad 65 in which the protective layer is connected to the backing layer or in which the backing layer is used as a protective layer has no components which have to be removed and disposed of in connection with the use of the incontinence pad 65, because the protective layer 70 constitutes an integrated part of the incontinence pad 65.

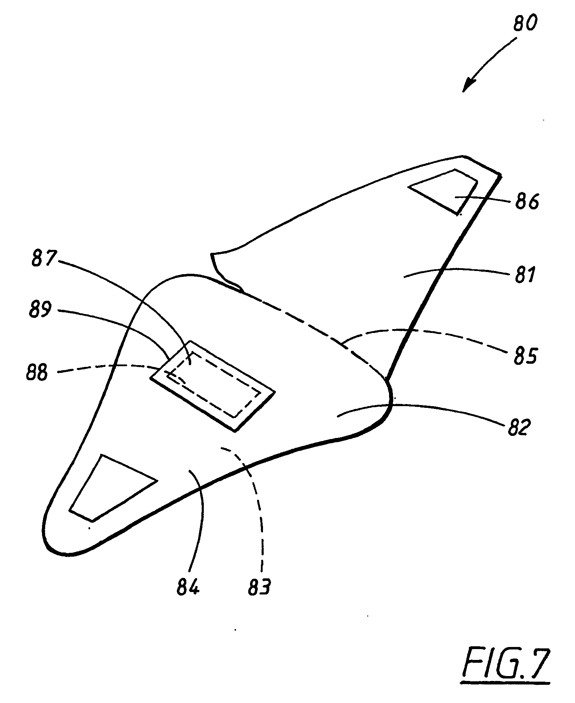

Figure 7 shows a fifth embodiment of the invention. The figure shows the incontinence pad 80 from the side intended to face away from the wearer during use.

The incontinence pad 80 has a fixing tongue 81 which is connected removably to the absorbent part 82 of the incontinence pad 80. The fixing tongue 81 consists of an extension of the liquid-permeable surface layer 83 and backing layer 84 of the absorbent part 82, the fixing tongue having been made removable by virtue of a perforation 85 being arranged on the dividing line between the absorbent part 82 and the fixing tongue 81 of the incontinence pad 80. Alternative methods of arranging a removable connection between the absorbent part 82 and the fixing tongue 81 such as, for example, a break-open thermal weld or ultrasonic weld, openable connecting glue or the like are also conceivable.

In Figure 7, the perforation 85 has been partly tom apart in order to illustrate how the fixing tongue 81 can be removed from the absorbent part 82 of the incontinence pad 80.

The advantage of a removable fixing tongue 81 is that the wearer of the incontinence pad 80 can choose whether he wants to fix the incontinence pad 80 to his underpants by means of the fixing tongue 81 or if he only wants to use the absorbent part 82 of the incontinence pad 80 and fix this to the crotch portion of the underpants.

The incontinence pad 80 comprises a first fastening means 86 arranged on the fixing tongue 81 and a second fastening means 87 arranged on the backing layer 84 of the absorbent part 82 and intended to be fixed to the crotch portion of the underpants of the wearer.

The second fastening means 87 is arranged so that the wearer of the incontinence pad 80 can choose whether he wants to make use of the second fastening means 87 by activating the same before use of the incontinence pad 80 or if he wants to use the incontinence pad 80 without activating the second fastening means 87. The activatable second fastening means 87 comprises a pressure-sensitive adhesive surface 88 which has been covered by a removable protective layer 89, activation being effected by removal of the protective layer 89.

The first fastening means 86 is arranged in the same way as the fastening means 69 according to the fourth embodiment described above and in Figures 5 and 6.

An incontinence pad 80 comprising a removable fixing tongue 81 and an activatable second fastening means 87 arranged on the absorbent part 82 of the incontinence pad 80 means that the wearer has a number of possibilities for choosing how the incontinence pad 80 is to be fixed during use.

The wearer can choose to fix the incontinence pad 80 via only the fixing tongue 81, in which case the wearer chooses not to activate the second fastening means 87 but opts instead to allow the protective layer 89 to remain over the pressure-sensitive adhesive surface 88.

If the wearer wants to fix the incontinence pad 80 by means of both the first fastening means 86 and the second fastening means 87, the wearer opts not to remove the fixing tongue 81, but to activate the second fastening means 87 by removing the protective layer 89, in which case the incontinence pad 80 will be fixed both to the crotch portion of the underpants and via the fixing tongue 81.

If the wearer only wants to fix the incontinence pad 80 to the crotch portion of the underpants, he removes the fixing tongue 81 by tearing the fixing tongue 81 off along the perforation 85. He can then choose whether he wants to attach the incontinence pad 80 to the underpants by means of the pressure-sensitive adhesive surface 88 of the second fastening means 87 by activating this or simply to allow the incontinence pad 80 to be held in place by the crotch portion of the underpants. If the wearer chooses simply to allow the incontinence pad 80 to be held in place by the crotch portion of the underpants, he does not activate the second fastening means 87, that is to say he allows the protective layer 89 to remain on the incontinence pad 80.

The invention also includes all conceivable combinations of the illustrative embodiments described.

The invention is furthermore not limited to the illustrative embodiments mentioned above, but can of course be applied to other embodiments within the scope of the patent claims below.

## Claims

1. Disposable incontinence pad (1; 35; 50; 65; 80) with a longitudinal direction and a transverse direction and intended for men, and also comprising an absorbent part (5; 37; 53; 67; 82) with an upper liquid-permeable surface layer (2; 40; 83), a lower backing layer (3; 41; 68; 84), and an absorbent body (4) arranged between the liquid-permeable surface layer (2; 40; 83) and the backing layer (3; 41; 68; 84), and also having a front end portion (23; 38; 54), the Incontinence pad (1; 35; 50; 65; 80) being provided with a fastening system for fixing the incontinence pad (1: 35; 50; 65; 80) to a pair of underpants, fixing pants or the like, **characterized in that** the fastening system comprises at least one fixing tongue (15; 36; 51; 66; 81) extending in the longitudinal direction of the incontinence pad (1; 35; 50; 65: 80) from the front end portion (23; 38; 54) only.

2. Incontinence pad (1) according to Claim 1, **characterized in that** the fixing tongue (15) consists of an extension of at least one of the liquid-permeable surface layer (2) and the backing layer (3).

3. Incontinence pad (1) according to Claim 1 or 2, **characterized in that** the fixing tongue comprises a thin absorbent material layer.

4. Incontinence pad (35) according to Claim 1, 2 or 3, **characterized in that** the entire fixing tongue (36) comes to lie inside the edges of the absorbent part (37) of the incontinence pad (35) when the entire fixing tongue (36) is folded over the liquid-permeable surface layer (40) or the backing layer (41).

5. Incontinence pad (1) according to any one of Claims 1-4, **characterized in that** the fixing tongue (15) comprises at least one fastening means (16) intended to be fixed to fixing pants during use.

6. Incontinence pad (1) according to Claim 5, **characterized in that** the fastening means (16) is arranged to be fixed to the inside of the underpants of the wearer in the area between the crotch portion and the waist of the underpants.

7. Incontinence pad (1) according to any one of Claims 1-4, **characterized in that** the fixing tongue (15) is arranged to be folded around the waist of a pair of underpants, fixing pants or the like, the waist elastic of the underpants or fixing pants fixing the incontinence pad (1) via the fixing tongue (15).

8. Incontinence pad (1) according to Claim 7, **characterized in that** the fixing tongue (15) comprises a fastening means (16) arranged to be fixed to the outside of the fixing pants or underpants.

9. Incontinence pad (65) according to Claim 5, 6 or 8, **characterized in that** the fastening means (68) comprises a pressure-sensitive adhesive surface (71), and **in that** a removable protective layer (70) is arranged over the pressure-sensitive adhesive surface (71), the protective layer (70) being connected to the backing layer (68) of the incontinence pad.

10. Incontinence pad (65) according to Claim 5, 6 or 8, **characterized in that** at least one area of the backing layer (68) is treated with a release agent, and **in that** the fastening means (69) comprises a pressure-sensitive adhesive surface (71), the pressure-sensitive adhesive surface (71) being connected removably to the release-agent-treated area.

11. . Incontinence pad (65) according to Claim 5, 6 or 8, **characterized in that** the fastening means (69) comprises a hook and loop surface, and **in that** a removable protective layer (70) is arranged over the hook and loop surface, the protective layer (70) being connected to the backing layer (68) of the incontinence pad (65).

12. incontinence pad (35) according to Claim 1, **characterized in that** the fixing tongue (36) consists of a band.

13. Incontinence pad (50) according to Claim 1, **characterized in that** the incontinence pad (50) comprises two fixing tongues (51; 52).

14. Incontinence pad (80) according to any one of the preceding claims, **characterized in that** the fixing tongue (81) can be separated from the absorbent part (82) of the incontinence pad (80).

15. Incontinence pad (80) according to any one of the preceding claims, **characterized in that** the incontinence pad (80) comprises a second fastening means (86) arranged on the backing layer (84) of the absorbent part (82), a removable protective layer (89) being arranged over the second fastening means (86).

16. Incontinence pad (1; 35; 50; 65; 80) according to any one of the preceding claims, **characterized in that** the absorbent part (5; 37; 53; 67; 82) of the incontinence pad has its greatest extent in the transverse direction in the part which, in the longitudinal direction, is located towards the fixing tongue (15; 36; 51; 66; 81) and its smallest extent in the part which, in the longitudinal direction, is located away from the fixing tongue.

## Patentansprüche

1. Einweginkontinenzpad (1; 35; 50; 65; 80) mit einer Längsrichtung und einer Querrichtung, das für Männer gedacht ist, und ferner umfassend einen Absorptionsteil (5; 37; 53; 67; 82) mit einer oberen flüssigkeitsdurchlässigen Oberflächenlage (2; 40; 83), einer unteren Decklage (3; 41; 68; 84) und einem zwischen der flüssigkeitsdurchlässigen Oberflächenlage (2; 40; 83) und der Decklage (3; 41; 68; 84) angeordneten Absorptionskörper (4), und weiter aufweisend einen Vorderendabschnitt (23; 38; 54), wobei das Inkontinenzpad (1; 35; 50; 65; 80) mit einem Befestigungssystem zum Befestigen des Inkontinenzpads (1; 35; 50; 65; 80) an einer Unterhose, einem Befestigungshöschen oder ähnlichem versehen ist, **dadurch gekennzeichnet, dass** das Befestigungssystem wenigstens eine Befestigungszunge (15; 36; 51; 66; 81) umfasst, die sich nur von dem Vorderendabschnitt (23; 38; 54) in der Längsrichtung des Inkontinenzpads (1; 35; 50; 65; 80) erstreckt.

2. Inkontinenzpad (1) nach Anspruch 1, **dadurch gekennzeichnet, dass** die Befestigungszunge (15) aus einem Fortsatz von wenigstens der flüssigkeitsdurchlässigen Oberflächenlage (2) oder der Decklage (3) besteht.

3. Inkontinenzpad (1) nach Anspruch 1 oder 2, **dadurch gekennzeichnet, dass** die Befestigungszunge eine dünne Absorptionsmateriallage umfasst.

4. Inkontinenzpad (35) nach Anspruch 1, 2 oder 3, **dadurch gekennzeichnet, dass** die gesamte Befestigungszunge (36) innerhalb der Kanten des Absorptionsteils (37) des Inkontinenzpads (35) zu liegen kommt, wenn die gesamte Befestigungszunge (36) auf die flüssigkeitsdurchlässige Oberflächenlage (40) oder die Decklage (41) geklappt ist.

5. Inkontinenzpad (1) nach einem der Ansprüche 1 bis 4, **dadurch gekennzeichnet, dass** die Befestigungszunge (15) wenigstens eine Befestigungseinrichtung (16) umfasst, die dazu gedacht ist, im Gebrauch an einem Befestigungshöschen befestigt zu werden.

6. Inkontinenzpad (1) nach Anspruch 5, **dadurch gekennzeichnet, dass** die Befestigungseinrichtung (16) derart angeordnet ist, dass sie an der Innenseite der Unterwäsche des Trägers im Bereich zwischen dem Schrittabschnitt und der Taille der Unterwäsche zu befestigen ist.

7. Inkontinenzpad (1) nach einem der Ansprüche 1 bis 4, **dadurch gekennzeichnet, dass** die Befestigungszunge (15) derart angeordnet ist, dass sie um die Taille der Unterhose, des Befestigungshöschens oder ähnlichem zu klappen ist, wobei die Taillenelastik der Unterhose oder des Befestigungshöschens das Inkontinenzpad (1) über die Befestigungszunge (15) fixiert.

8. Inkontinenzpad (1) nach Anspruch 7, **dadurch gekennzeichnet, dass** die Befestigungszunge (15) eine Befestigungseinrichtung (16) umfasst, die derart angeordnet ist, das sie an der Außenseite des Befestigungshöschens oder der Unterhose zu befestigen ist.

9. Inkontinenzpad (1) nach Anspruch 7, 6 oder 8, **dadurch gekennzeichnet, dass** die Befestigungseinrichtung (68) eine Haftklebemitteloberfläche (71) umfasst, und dadurch, dass eine entfernbare Schutzschicht (70) über der Haftklebemitteloberfläche (71) angeordnet ist, wobei die Schutzschicht (70) mit der Decklage (68) des Inkontinenzpads verbunden ist.

10. Inkontinenzpad (65) nach Anspruch 5, 6 oder 8, **dadurch gekennzeichnet, dass** wenigstens ein Bereich der Decklage (68) mit einem Trennmittel behandelt ist und dadurch, dass die Befestigungseinrichtung (69) eine Haftklebemittelfläche (71) umfasst, wobei die Haftklebemittelfläche (71) lösbar mit dem mit Trennmittel behandelten Bereich verbunden ist.

11. Inkontinenzpad (65) nach Anspruch 5, 6 oder 8, **dadurch gekennzeichnet, dass** die Befestigungseinrichtung (69) eine Klettverschlussfläche umfasst und dadurch, dass eine entfernbare Schutzschicht (70) über der Klettverschlussfläche angeordnet ist, wobei die Schutzschicht (70) mit der Decklage (68) des Inkontinenzpads verbunden ist.

12. Inkontinenzpad (35) nach Anspruch 1, **dadurch gekennzeichnet, dass** die Befestigungszunge (36) aus einem Streifen besteht.

13. Inkontinenzpad (50) nach Anspruch 1, **dadurch gekennzeichnet, dass** das Inkontinenzpad (50) zwei Befestigungszungen (51, 52) umfasst.

14. Inkontinenzpad (80) nach einem der vorstehenden Ansprüche, **dadurch gekennzeichnet, dass** die Befestigungszunge (81) von dem Absorptionsteil (82) des Inkontinenzpads (80) abgetrennt werden kann.

15. Inkontinenzpad (80) nach einem der vorstehenden Ansprüche, **dadurch gekennzeichnet, dass** das Inkontinenzpad (80) eine zweite Befestigungseinrichtung (86) umfasst, die auf der Decklage (84) des Absorptionsteils (82) angeordnet ist, wobei eine entfernbare Schutzschicht (89) über der zweiten Befestigungseinrichtung (86) angeordnet ist.

16. Inkontinenzpad (1; 35; 50; 65; 80) nach einem der vorstehenden Ansprüche, **dadurch gekennzeichnet, dass** der Absorptionsteil (5; 37; 53; 67; 82) des Inkontinenzpads in der Querrichtung seine größte Erstreckung in dem Teil aufweist, der in der Längsrichtung in Richtung der Befestigungszunge (15; 36; 51; 66; 81) angeordnet ist, und seine geringste Erstreckung in dem Teil, der in der Längsrichtung entfernt der Befestigungszunge angeordnet ist.

## Revendications

1. Protection contre l'incontinence jetable (1 ; 35 ; 50 ; 65 ; 80) ayant une direction longitudinale et une direction transversale et destinée aux hommes, et comprenant aussi une partie absorbante (5 ; 37 ; 53 ; 67 ; 82) ayant une couche de surface supérieure (2 ; 40 ; 83) perméable aux liquides, une couche de renfort arrière (3 ; 41 ; 68 ; 84) et un corps absorbant (4) placé entre la couche de surface supérieure (2 ; 40 ; 83) perméable aux liquides et la couche de renfort (3 ; 41 ; 68 ; 84), et comportant aussi une partie d'extrémité avant (23 ; 38 ; 54), la protection contre l'incontinence (1 ; 35 ; 50 ; 65 ; 80) étant pourvue d'un système de fixation pour fixer la protection contre l'incontinence (1 ; 35 ; 50 ; 65 ; 80) à un slip, à une culotte de fixation ou à un sous-vêtement similaire, **caractérisée en ce que** le système de fixation comprend au moins une languette de fixation (15 ; 36 ; 51 ; 66 ; 81) qui s'étend dans la direction longitudinale de la protection contre l'incontinence (1 ; 35 ; 50 ; 65 ; 80) seulement depuis la partie d'extrémité avant (23 ; 38 ; 54).

2. Protection contre l'incontinence (1) selon la revendication 1, **caractérisée en ce que** la languette de fixation (15) consiste en un prolongement d'au moins une couche parmi la couche de surface (2) perméable aux liquides et la couche de renfort (3).

3. Protection contre l'incontinence (1) selon la revendication 1 ou 2, **caractérisée en ce que** la languette de fixation comprend une mince couche de matériau absorbant.

4. Protection contre l'incontinence (35) selon la revendication 1, 2 ou 3, **caractérisée en ce que** la languette de fixation entière (36) se trouve à l'intérieur des bords de la partie absorbante (37) de la protection contre l'incontinence (35) lorsque la languette de fixation entière (36) est pliée sur la couche de surface (40) perméable aux liquides ou la couche de renfort (41).

5. Protection contre l'incontinence (1) selon l'une quelconque des revendications 1 à 4, **caractérisée en ce que** la languette de fixation (15) comprend au moins un moyen de fixation (16) destiné à être fixé à une culotte de fixation en utilisation.

6. Protection contre l'incontinence (1) selon la revendication 5, **caractérisée en ce que** le moyen de fixation (16) est adapté pour être fixé à l'intérieur du slip de l'utilisateur dans la zone située entre la partie d'entrejambe et la taille du slip.

7. Protection contre l'incontinence (1) selon l'une quelconque des revendications 1 à 4, **caractérisée en ce que** la languette de fixation (15) est adaptée pour être pliée autour de la taille d'un slip, d'une culotte de fixation ou similaire, l'élastique de taille du slip ou de la culotte de fixation permettant la fixation de la protection contre l'incontinence (1) via la languette de fixation (15).

8. Protection contre l'incontinence (1) selon la revendication 7, **caractérisée en ce que** la languette de fixation (15) comprend un moyen de fixation (16) adapté pour être fixé à l'extérieur de la culotte de fixation ou du slip.

9. Protection contre l'incontinence (65) selon la revendication 5, 6 ou 8, **caractérisée en ce que** le moyen de fixation (68) comprend une surface adhésive sensible à la pression (71), et **en ce qu'**une couche protectrice amovible (70) est placée sur la surface adhésive sensible à la pression (71), la couche protectrice (70) étant connectée à la couche de renfort (68) de la protection contre l'incontinence.

10. Protection contre l'incontinence (65) selon la revendication 5, 6 ou 8, **caractérisée en ce qu'**au moins une zone de la couche de renfort (68) est traitée avec un agent anti-adhésif, et **en ce que** le moyen de fixation (69) comprend une surface adhésive sensible à la pression (71), la surface adhésive sensible à la pression (71) étant connectée de manière amovible à la zone traitée à l'agent anti-adhésif.

11. Protection contre l'incontinence (65) selon la revendication 5, 6 ou 8, **caractérisée en ce que** le moyen de fixation (69) comprend une surface à crochets et à boucles, et **en ce qu'**une couche protectrice amovible (70) est placée sur la surface à crochets et à boucles, la couche protectrice (70) étant connectée à la couche de renfort (68) de la protection contre l'incontinence (65).

12. Protection contre l'incontinence (35) selon la revendication 1, **caractérisée en ce que** la languette de fixation (36) est constituée d'une bande.

13. Protection contre l'incontinence (50) selon la revendication 1, **caractérisée en ce que** la protection contre l'incontinence (50) comprend deux languettes de fixation (51 ; 52).

14. Protection contre l'incontinence (80) selon l'une quelconque des revendications précédentes, **caractérisée en ce que** la languette de fixation (81) peut être séparée de la partie absorbante (82) de la protection contre l'incontinence (80).

15. Protection contre l'incontinence (80) selon l'une quelconque des revendications précédentes, **caractérisée en ce que** la protection contre l'incontinence (80) comprend un deuxième moyen de fixation (86) placé sur la couche de renfort (84) de la partie absorbante (82), une couche protectrice amovible (89) étant placée sur le deuxième moyen de fixation (86).

16. Protection contre l'incontinence (1 ; 35 ; 50 ; 65 ; 80) selon l'une quelconque des revendications précédentes, **caractérisée en ce que** la partie absorbante (5 ; 37 ; 53 ; 67 ; 82) de la protection contre l'incontinence a sa plus grande dimension dans la direction transversale dans la partie qui, dans la direction longitudinale, est située vers la languette de fixation (15 ; 36 ; 51 ; 66 ; 81) et sa plus petite dimension dans la partie qui, dans la direction longitudinale, est située à l'écart de la languette de fixation.
